Europäisches Patentamt

European Patent Office

Office européen des brevets

(11) Veröffentlichungsnummer: **0 302 834**
**A2**

## (12) EUROPÄISCHE PATENTANMELDUNG

(21) Anmeldenummer: 88810536.8

(22) Anmeldetag: 05.08.88

(51) Int. Cl.⁴: **A 61 B 5/08**
**A 61 M 15/00, A 61 K 49/02**

(30) Priorität: 07.08.87 CH 3042/87

(43) Veröffentlichungstag der Anmeldung:
08.02.89 Patentblatt 89/06

(84) Benannte Vertragsstaaten:
BE CH DE FR GB LI NL SE

(71) Anmelder: **Solco Basel AG**
**Gellertstrasse 18**
**CH-4052 Basel (CH)**

(72) Erfinder: **Waser, John**
**Leimenstrasse 18**
**CH-4051 Basel (CH)**

(74) Vertreter: **Eschmann, Heinz et al**
**A. Braun, Braun, Héritier, Eschmann AG Patentanwälte**
**Holbeinstrasse 36-38**
**CH-4051 Basel (CH)**

(54) **Verfahren zur Erzeugung von Aerosolen für die szintigraphische Messung der Lungenventilation sowie diesbezügliche Vorrichtung.**

(57)  Gemäss dem vorliegenden Verfahren zur Erzeugung von Aerosolen zur szintigraphischen Erfassung der regionalen Lungenventilation wird eine radioaktiv markierte Substanz, vorzugsweise ein Tc-99m-Komplex, in eine Vernebelungskammer (5) eingebracht und mit einem Ultraschallvibrator (3) vernebelt. Als Trägersubstanz in der radioaktiv markierten Substanz wird ein Kolloid aus Humanserumalbumin verwendet, das eine unregelmässige Oberflächenstruktur bei dennoch annähernd uniformer Partikelgrösse besitzt. Das so erzeugte Aerosol wird durch einen Inhalationsschlauch (9) abgeatmet.

Die Wandung der Vernebelungskammer (5) ist wenigstens teilweise als Schallmembran ausgebildet und weist eine Wandstärke zwischen 0.02 mm und 0.03 mm auf.

FIG.1

EP 0 302 834 A2

**Beschreibung**

**Verfahren zur Erzeugung von Aerosolen für die szintigraphische Messung der Lungenventilation sowie diesbezügliche Vorrichtung**

Die vorliegende Erfindung betrifft ein Verfahren gemäss Oberbegriff des Patentanspruches 1 sowie eine diesbezügliche Vorrichtung.

Ventilation und Perfusion (Durchblutung) sind neben der Diffusion die wichtigsten Teilkomponenten der Lungenfunktion.

Zur Zeit sind zwei szintigraphische Verfahren zur Messung der regionalen Lungenventilation bekannt, nämlich die Ventilationsszintigraphie einerseits und die Inhalationszintigraphie andererseits. In beiden Fällen werden radioaktive Gase oder Partikel mit möglichst kurzer Halbwertszeit inhaliert und die Radioaktivität in der Lunge mit einer Gammastrahlen-Kamera, einem Scanner oder einem anderen geeigneten Detektor erfasst und ausgewertet.

Bei der Ventilationsszintigraphie wird bevorzugt Krypton-81m eingesetzt. Dieses weist eine sehr kurze physikalische Halbwertszeit (13 Sek.) auf. Grösster Nachteil ist dessen häufig mangelnde Verfügbarkeit, so dass dieses Radioisotop gerade für die in der Praxis wichtige Lungenembolie-Diagnostik wenig geeignet ist. Ferner wirkt sich aber auch die aufwendige Handhabung, auf die wir später noch zu sprechen kommen, nachteilig aus.

Für die Inhalationsszintigraphie werden Aerosole eingesetzt. Ein Aerosol setzt sich aus einem Gas und feinstverteilten festen oder flüssigen Schwebestoffen zusammen.

Idealerweise sollten nur monodisperse (Partikel gleicher Grösse aufweisende) Radioaerosole zum Einsatz kommen. Bei polydispersen Radioaerosolen kann ein kleiner Anteil grösserer Tröpfchen einen Grossteil der Aktivität beinhalten, da der Radius in dritter Potenz in das Volumen eines kugelförmigen Partikels eingeht. Die Partikelgrösse sollte sich innerhalb eines vorgegebenen Bereiches bewegen, damit die Gleichmässigkeit der Ablagerung des Aerosols in der Lunge gewährleistet ist. Andernfalls kann keine zuverlässige Diagnose gestellt werden.

Beispielsweise sind in der PCT-Anmeldung WO 83/03342 ein Verfahren und eine Vorrichtung angegeben, bei welchen mittels Pressluft ein Aerosol erzeugt wird.

Nachteilig ist die Abhängigkeit von einem Druckgassystem. Die üblichen nachfüllbaren Behälter machen einen hohen adminis trativen Aufwand erforderlich, da sie dem Kontrollbereich des Strahlenschutzes unterstellt sind. Als Alternative bietet sich das spitaleigene Druckluftsystem. Doch dies ist auch nicht unproblematisch, da dessen Druck in der Regel starken Schwankungen unterworfen ist, so dass zumindest mit den bisher bekannten Zerstäubern eine minimal erforderliche Monodispersität des erzeugten Aerosols nicht gewährleistet ist.

Aus der US-Patentschrift 4,094,317 ist eine Vorrichtung bekannt, bei welcher ein Aerosol mittels Ultraschall erzeugt werden kann. Eine Vernebelungskammer enthält in ihrem unteren Bereich die zur Bildung des Aerosols bestimmte, radioaktiv markierte Substanz. Diese enthält eine mit einem radioaktiven Marker markierte Trägersubstanz. Der die radioaktiv markierte Substanz enthaltende untere Kammerbereich ist in einen Behälter eingetaucht, der den Ultraschallgenerator enthält und mit einem Ultraschall leitenden Medium gefüllt ist. Es wird ein Aerosol erzeugt, dessen Tröpfchen in ihrer Grösse stark variieren. Eine Prallkugel dient dazu, die Tröpfchen, die eine Grösse von 3.5 Mikrometer übersteigen, abzufangen. Die sich hierbei im unteren Bereich der Prallkugel ansammelnde Flüssigkeit tropft wieder in die radioaktiv markierte Flüssigkeit im Boden der Vernebelungskammer zurück und kann wieder verwendet werden. Abgesehen davon, dass es fraglich ist, ob die Prallkugel genügt, um die Tröpfchen, deren Grösse die oben angegebene Grenze übersteigt, in ausreichendem Masse abzufangen, ist die genannte Grenze für die Anwendung in der Lungenszintigraphie gemäss einer viel beachteten Studie von Taplin, gemäss welcher die Grösse der Partikel 2 Mikrometer nicht übersteigen sollte, bereits zu hoch (George V. Taplin: "Lung perfusion-inhalation, scintigraphy in obstructive airway disease and pulmonary embolism" in "Radiologic Clinics of North America" - Vol XVI. No. 3, December 1978).

Weitere Vorrichtungen zur Erzeugung eines Aerosols mittels Ultraschall sind aus der DE-OS 1 813 776 und der französischen Patentschrift Nr. 72.42989 bekannt. In beiden Fällen sind die Vorrichtungen unter anderem für eine Inhalationstherapie konzipiert. Infolgedessen ist eine höhere Obergrenze für die Partikelgrösse zulässig (gemäss der genannten DE-OS 6 Mikrometer), so dass die Geräte für die Lungenszintigraphie nicht geeignet sind.

In den zitierten Schriften und in der übrigen uns bekannten Literatur sind keine für die Anwendungen in der Lungenszintigraphie geeigneten Massnahmen offenbart, mittels welchen die Obergrenze der Partikelgrösse bei Ultraschallverfahren reduziert werden könnte. Filtermassnahmen, mittels welchen die zu grossen Partikel zuverlässig abgefangen werden könnten, erzeugen nämlich einen beträchtlichen Luftwiderstand. Gerade schwer lungengeschädigte Patienten, beispielsweise mit chronisch obstruktiven Lungenerkrankungen, mit Pulmonalembolien oder Bronchialkarzinomen, bei denen die szintigraphischen Untersuchungen von besonderer Bedeutung sind, können das Aerosol nicht in einem für eine Diagnose ausreichendem Masse inhalieren.

Ein weiteres Problem zeigte sich bei der Auswahl des geeigneten Trägermediums für den radioaktiven Marker. Es zeigte sich nämlich, dass die Ultraschallenergie bei den meisten Trägermedien die Bindung des radioaktiven Markers an den Trägerpartikel löst, so dass das Austreten von freien radioaktiven Markern zu falschen klinischen Resultaten führt.

Schliesslich ist noch auf die Problematik hinsichtlich Aktivität der für die zur Erzeugung des Aerosols benötigten radioaktiven Substanz hinzuweisen. Die erforderliche Mindestaktivität ist bei den bisher bekannten Vorrichtungen verhältnismässig hoch (gemäss der genannten US-PS 4,094,317 wird 20-30 mCi. für annähernd 0.5 ccm Ausgangssubstanz benötigt).

Es ist Aufgabe der Erfindung, ein Verfahren anzugeben, mittels welchem bei möglichst geringer Aktivität der radioaktiv markierten Substanz eine für die Lungenszintigraphie ausreichende Aerosolmenge mit ausreichender Aktivitätskonzentration und dennoch ausreichender Bindungskraft der Markierungssubstanz an den Trägerpartikel erzeugt werden kann.

Diese Aufgabe wird entsprechend dem kennzeichnenden Teil des Patentanspruches 1 gelöst.

Eine weitere Aufgabe der Erfindung ist es, eine Vorrichtung zu schaffen, mittels welcher mit möglichst einfachen Mitteln ein ausreichend monodisperses Aerosol generiert werden kann, dessen Tröpfchengrösse 2 Mikrometer nicht übersteigt und dennoch dem Atmungsvorgang einen möglichst geringen Widerstand entgegensetzt.

Es ist ferner Aufgabe der Erfindung, ein Verfahren anzugeben, mittels welchem eine leichte Entsorgung von billigen Einmalelementen möglich ist, so dass der administrative und technische Aufwand unter Einhaltung sämtlicher Strahlenschutzvorschriften möglichst gering gehalten werden kann.

Diese Aufgaben werden gemäss dem kennzeichenden Teil des Vorrichtungsanspruches 3 gelöst. Bevorzugte Ausführungsformen ergeben sich aus den Unteransprüchen.

Wie schon oben erwähnt, besteht bei den herkömmlichen radioaktiv markierten Substanzen die Tendenz, dass sich die Markierungssubstanz beim Vernebelungsvorgang von den Trägerpartikeln löst. Dies dürfte nach unseren Untersuchungen darauf zurückzuführen sein, dass die herkömmlichen radioaktiv markierten Substanzen annähernd sphärische Trägerpartikel mit weitgehend glatter Oberfläche verwenden.

Erfindungsgemäss wird ein Kolloid aus Humanserumalbumin verwendet, das eine unregelmässige Oberflächenstruktur bei dennoch annähernd uniformer Partikelgrösse besitzt. Diese Eigenschaften werden beispielsweise von einem gemäss der französischen Patentanmeldung Nr. 82 14722 beschriebenen Verfahren hergestellten Kolloid aus Humanserumalbumin (HSA), welches sich unter anderem für die Lungenperfusionsmessungen bewährt hat, in hervorragendem Masse erfüllt. Das Kolloid wird unter dem Namen "SOLCO VENTICOLL®" vertrieben. Als radioaktiver Marker wird hierzu Technetium-99m (Tc-99m) verwendet.

Messungen haben gezeigt, dass eine Aktivität der radioaktiv markierten Substanz von 370 bis 555 MBq (10 bis 15 mCi) schon ausreichend ist, um ein Lungenszintigramm durchzuführen. Infolgedessen kann mit einem aus einem Generator, der eine sehr geringe Aktivität oder Restaktivität aufweist, im Gegensatz zu den bisher bekannten Verfahren noch gearbeitet werden.

Ein weiterer Vorteil der Erfindung ist es, dass nur Wegwerfteile bakteriell und radioaktiv kontaminiert werden. Der Vibrator bleibt in der Abschirmung. Der Stromgenerator wird nicht kontaminiert und muss deshalb keinen besonderen Sicherheitsmassnahmen unterworfen werden. Stromanschluss ist an Orten, an welchen solche Untersuchungen durchgeführt werden, in den meisten Fällen vorhanden. Umtriebe, beispielsweise mit zu wenig gefüllten Druckluftflaschen, werden vermieden.

Nachstehend wird die Erfindung anhand einer Zeichnung beispielsweise näher erläutert. Es zeigen:

    Fig. 1 eine schematische Darstellung eines Aerosol-Generators;

    Fig. 2 eine Darstellung der luftleitenden Wege;

    Fig. 3 eine Detaildarstellung des Prallplattenaggregates und

    Fig. 4 eine Detaildarstellung des Einfüllstutzens.

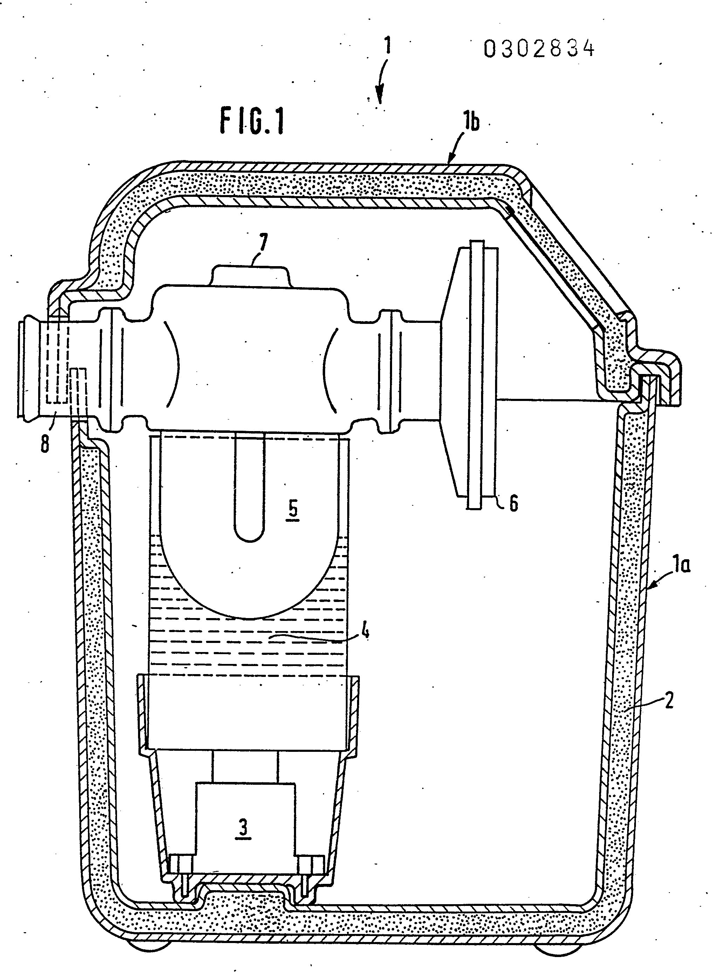

In Figur 1 ist ein Aerosolgenerator dargestellt. Das Gehäuse 1 ist zweiteilig aufgebaut und besteht aus einem die Generatorelemente enthaltenden unteren Teil 1a und einem als abnehmbarer Deckel dienenden oberen Teil 1b. In der Gehäusewandung ist eine punktiert angedeutete Abschirmung 2 enthalten, welche beispielsweise aus Blei bestehen kann. Auf dem Boden des unteren Gehäuseteils 1a ist ein Ultraschallvibrator 3 abnehmbar montiert. Er wird über eine nicht näher darge stellte Steckverbindung von einem ebenfalls nicht dargestellten Stromgenerator mit einer Frequenz von vorzugsweise zwischen 2.1 und 2.7 MHz erregt. Als Vibratorelement kommt beispielsweise ein Piezokristall in Frage. Der Aufbau eines Ultraschallvibrators ist dem Fachmann bestens bekannt, so dass auf eine Erläuterung an dieser Stelle verzichtet werden kann.

Die vom Ultraschallvibrator 3 erzeugten Vibrationen werden via einer schallübertragenden Flüssigkeit 4 auf die Vernebelungskammer 5 übertragen. Der untere Teil dieser Vernebelungskammer 5 dient der Aufnahme der radioaktiv markierten Substanz und ist vorzugsweise in Form einer nach unten gerichteten Halbkugel ausgebildet. Der Durchmesser dieser Halbkugel sollte derart gewählt werden, dass letztere bei einem kleinen Volumen radioaktiver Substanz dennoch eine genügend grosse Aerosol abnebelnde Oberfläche aufweist. In der Praxis hat sich ein Durchmesser zwischen 40mm und 50 mm als geeignet erwiesen. Im oberen Teil des in das schallübertragende Medium 4 eintauchenden Teils der Vernebelungskammer 5 ist in der Figur eine Einbuchtung angedeutet. Solche Einbuchtungen dienen dem Entweichen allfälliger Gase, die sich infolge der einwirkenden Ultraschallenergie an der Grenzfläche zum schalleitenden Medium zu bilden pflegen und an der kugeligen Aussenseite der Vernebelungskammer hinaufwandern.

Als schallübertragende Flüssigkeit kann ohne weiteres Wasser verwendet werden. Auf diese Weise wird eine besonders gleichmässige Schallübertragung auf den die radioaktiv markierte Substanz enthaltenden Boden der Vernebelungskammer 5 bewirkt. Diese Flüssigkeit wird durch einen Einlassstutzen 7 in die Vernebelungskammer 5 eingebracht.

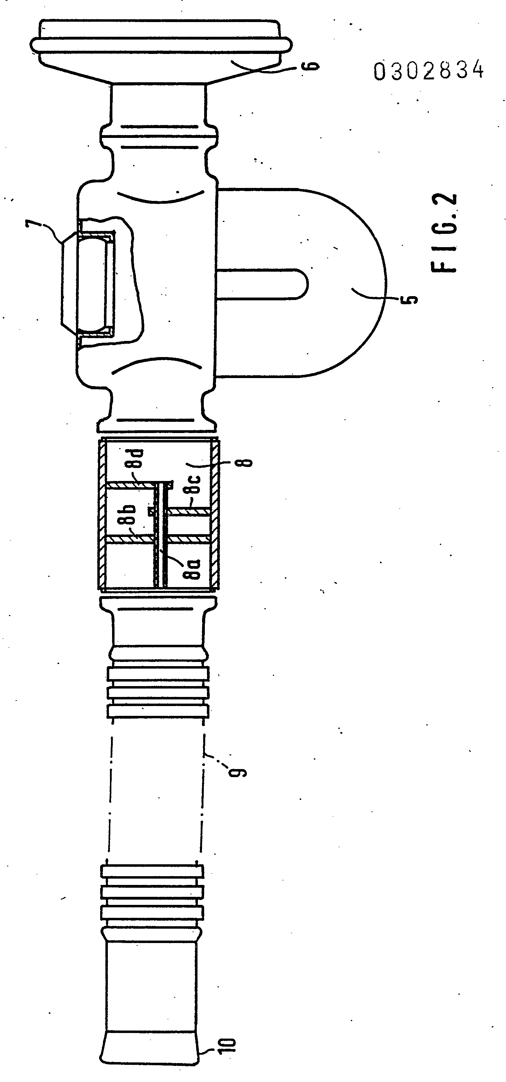

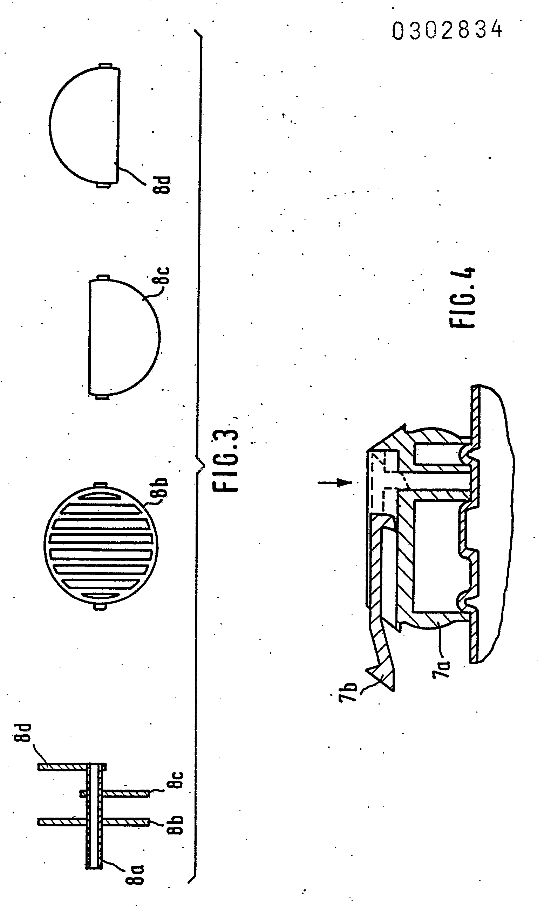

Das in der Vernebelungskammer 5 erzeugte Aerosol gelangt in ein Prallplattenaggregat 8. Dieses führt, wie in Figur 2 gezeigt ist, zu einem Inhalationsschlauch 9, an dessen Ende ein Mundstück 10 angebracht wird. Die Beschreibung des Prallplattenaggregates sowie die Erklärung der Ueberweisungsziffern 8a bis 8d erfolgt nun anhand der Detailzeichnungen in Figur 3. Drei Prallplatten 8b,8c und 8d sind auf einem Prallplattenhalter 8a befestigt. Die kreisscheibenförmige Prallplatte 8b ist flüssigkeitsdicht im Prallplattengehäuse 8 montiert. Sie weist mehrere Schlitze auf, die einen vorgegebenen Minimalabstand von der Gehäusewand haben, damit sich etwa ansammelndes Kondensat nicht in den Inhalationsschlauch gelangen kann. Die beiden anderen Prallplatten 8c und 8d weisen eine Halbkreisform auf und sind diametral versetzt montiert. Im Interesse eines möglichst geringen Atmungswiderstandes kann jedoch auch anstelle einer kreisscheibenförmigen Prallplatte 8b eine Prallplatte mit einem halbkreisförmigen Querschnitt verwendet werden. Eine solche halbkreisförmige Prallplatte 8d wäre dann wie die Prallplatte 8b nach oben ausgerichtet. Anzahl, Form, Grösse und Ausrichtung der Prallplatten kann je nach Ausführungsform variieren. Die diesbezügliche Gestaltung ist Sache des Fachmannes.

Figur 4 zeigt eine Detailzeichnung des in Figur 1 und 2 angedeuteten Einfüllstutzens 7. Ein Pfeil weist auf die Oeffnung des Einlassteiles 7a. Wenn nun die radioaktiv markierte Substanz eingelassen ist, wird ein Schieber 7b über die Oeffnung geschoben. Anschliessend sorgen die Widerhaken dafür, dass der Einlassstutzen 7 kontaminationsdicht bleibt.

Die inspirierte und die expirierte Luft gelangt durch ein Filter 6 (Figur 1). In der Figur nicht dargestellte Gehäuseschlitze dienen dem für die Atmung benötigten Luftaustausch. Derartige Schlitze können beispielsweise seitlich im Gehäuse vorgesehen sein. Sie sind vorteilhafterweise schräg zu führen, damit eine vollständige Abschirmung gewährleistet ist.

Das Verfahren wird wie folgt durchgeführt:

Die radioaktiv markierte Substanz, mit welcher das Aerosol erzeugt werden soll, wird in einer Menge von wenigstens 3 ml beispielsweise mit einer Spritze durch den Einlassteil 7a des Einfüllstutzens 7 in die Vernebelungskammer 5 eingefüllt. Anschliessend wird der Schieber 7b geschlossen. Die Wider-haken und poröse Oberflächenbeschaffenheit stel-len sicher, dass der Schieber 7b zerstörungsfrei nicht mehr geöffnet werden kann. Aus Sicherheitsgründen - zur Vermeidung bakterieller oder strah-lenmässiger Kontamination - soll eine Wiederver-wendung verhindert werden.

Infolge des Expirierens der zu untersuchenden Person werden nämlich nicht nur das Mundstück 10 samt Inhalationsschlauch 9, sondern auch das Prallplattenaggregat 8, die Vernebelungskammer 5 und der Luftfilter 6 kontaminiert. Die genannten Elemente 5 bis 9 sind deshalb miteinander verschweisst und zum Einmalgebrauch bestimmt.

Nachdem die Flüssigkeit aktiviert und in die Vernebelungskammer eingefüllt und der Schieber 7b geschlossen ist, nimmt die zu untersuchende Person das Mundstück 10 in den Mund. Der Stromgenerator wird eingeschaltet und der Ultraschallvibrator 3 zum Schwingen angeregt. Die schalleitende Flüssigkeit 4 überträgt die Ultraschallschwingungen auf die Schallmembran.

Die Schallmembran weist die Form einer Halbkugel auf. Die Halbkugelform erlaubt eine optimale Raum- und Schallnutzung. Es ist von grösster Bedeutung, dass die Membran genügend dünn ist. Untersuchungen haben nämlich gezeigt, dass die Grösse der mit Ultraschall erzeugten Partikel mit der Dicke der Schallmembran korreliert ist.

Bezüglich Membrandicke hat sich eine Dicke zwischen 0.01 mm und 0.1 mm , vorzugsweise zwischen 0.02 mm bis 0.03 mm als günstig erwiesen. Bei zu geringen Membranstärken hat man mit einer unerwünschten Blasenbildung zwischen schalleitender Flüssigkeit und Schallmembran zu kämpfen.

Als Material für die Schallmembran haben sich Kunststoffe, beispielsweise modifizierte Polyolefine, insbsondere modifizierte Polyethylene, vorzüglich bewährt. Die Vernebelungskammer 5 kann samt Schallmembran aus einem Stück geformt werden.

**Patentansprüche**

1. Verfahren zur Erzeugung von Radioaerosolen für die szintigraphische Erfassung der regionalen Lungenventilation, bei welchem eine radioaktiv markierte Substanz, die eine mit einem radioaktiven Marker markierte Trägersubstanz enthält, in eine Vernebelungskammer eines mit Ultraschall arbeitenden Verneblers eingebracht und mittels Ultraschallenergie vernebelt wird, so dass auf diese Weise ein Aerosol erzeugt wird, bei welchem Verfahren ein Mundstück eines mit dem Vernebler verbundenen Inhalationsschlauches in den Mund genommen und das Aerosol während einer vorgegebenen Zeitdauer bzw. mit einer vorgegebenen Anzahl von Atemzügen inhaliert wird, dadurch gekennzeichnet, dass als Trägersubstanz für den radioaktiven Marker ein Kolloid aus menschlichem Humanserum verwendet wird, das eine unregelmässige Oberflächenstruktur bei dennoch annähernd uniformer Partikelgrösse besitzt.

2. Verfahren nach Anspruch 1, dadurch gekennzeichnet, dass als radioaktiver Marker Technetium 99m und als Trägersubstanz SOLCO VENTICOLL® verwendet wird.

3. Vorrichtung zur Erzeugung von Radioaerosolen für die szintigraphische Erfassung der regionalen Lungenventilation, mit einer in einem strahlengeschützten Behälter befindlichen Vernebelungskammer, an welche ein Luftfilter und ein mit einem Mundstück versehener Inhalationsschlauch angeschlossen ist, wobei der untere Bereich der Vernebelungskammer (5) mit einer radioaktiv markierten Substanz, die eine mit einem radioaktiven Marker markierte Trägersubstanz enthält, gefüllt ist, und wobei in diesem unteren Bereich die Wandung der Vernebelungskammer (5) wenigstens teilweise als Schallmembran ausgebildet und ein Ultraschallvibrator (3) vorhanden ist, mittels welchem über eine schalleitende Flüssigkeit (4) die Schallmembran in Schwingung versetzt wird, dadurch gekennzeichnet, dass als Trägersubstanz für den radioaktiven Marker ein Kolloid aus menschlichem Humanserum verwendet wird, das eine unregelmässige Oberflächenstruktur bei dennoch annähernd uniformer Partikelgrösse besitzt.

4. Vorrichtung nach Anspruch 3, dadurch gekennzeichnet, dass als Trägersubstanz SOLCO VENTICOLL® verwendet wird.

5. Vorrichtung nach einem der Ansprüche 3 oder 4, dadurch ge kennzeichnet, dass die als Schallmembran ausgebildete Wand der Vernebelungskammer (5) aus einem modifizierten Polyolefin, vorzugsweise einem modifizierten Polyethylen besteht, und dass die Wanddicke zwischen 0.02 mm und 0.03 mm beträgt.

6. Vorrichtung nach einem der Ansprüche 3 bis 5, dadurch gekennzeichnet, dass ein Ultraschallvibrator verwendet wird, der mit einer Frequenz von 2.1 MHz bis 2.7 MHz arbeitet.

7. Vorrichtung nach einem der Ansprüche 3 bis 6, dadurch gekennzeichnet, dass der Vernebelungskammer (5) ein Prallplattenaggregat (8a, 8b, 8c, 8d) nachgeschaltet ist.

FIG.1

0302834

FIG.2

8b 8d 8a 8c 8 10 9 5 6 7

0302834

8d

8a

8b 8c

8b

8c

8d

FIG.3

7b

7a

FIG.4